(19) Europäisches Patentamt
European Patent Office
Office européen des brevets

(11) EP 1 002 517 B1

(12) **FASCICULE DE BREVET EUROPEEN**

(45) Date de publication et mention de la délivrance du brevet:
**13.04.2005 Bulletin 2005/15**

(51) Int Cl.⁷: **A61K 7/13**

(21) Numéro de dépôt: **99402645.8**

(22) Date de dépôt: **25.10.1999**

(54) **Composition de teinture d'oxydation des fibres kératiniques et procédé de teinture mettant en oeuvre cette composition**

Oxidationsfärbemittel für keratinische Fäsern und Färbungsverfahren mit diesem Mittel

Composition for oxidative dyeing of keratinous fibres and dyeing process using same

(84) Etats contractants désignés:
**AT BE CH CY DE DK ES FI FR GB GR IE IT LI LU MC NL PT SE**

(30) Priorité: **20.11.1998 FR 9814654**

(43) Date de publication de la demande:
**24.05.2000 Bulletin 2000/21**

(73) Titulaire: **L'OREAL**
**75008 Paris (FR)**

(72) Inventeur: **Audousset, Marie-Pascale**
**92600 Asnieres (FR)**

(74) Mandataire: **Goulard, Sophie**
**L'OREAL-DPI**
**6 rue Bertrand Sincholle**
**92585 Clichy Cedex (FR)**

(56) Documents cités:
**DE-A- 19 637 371**
**DE-C- 19 534 214**
**GB-A- 2 260 135**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 002 517 B1

## Description

L'invention a pour objet une composition pour la teinture d'oxydation des fibres kératiniques, et en particulier des fibres kératiniques humaines telles que les cheveux comprenant, dans un milieu approprié pour la teinture, au moins un para-aminophénol substitué à titre de base d'oxydation, et du 1,3-bis-(β-hydroxyéthyl) amino 2-méthyl benzène et/ou au moins l'un de ses sels d'addition avec un acide à titre de coupleur, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet DE-A-4 132 615 des compositions pour la teinture d'oxydation des fibres kératiniques contenant un ou plusieurs dérivés de 2,6-diamino toluène à titre de coupleur, en association avec une ou plusieurs bases d'oxydation pouvant être choisies parmi les bases d'oxydation classiquement utilisées dans le domaine de la coloration d'oxydation telles que par exemple la paraphénylènediamine et le para-aminophénol non substitué.

Il a également déjà été proposé, notamment dans la demande de brevet DE-A-19 637 371, des compositions pour la teinture d'oxydation des fibres kératiniques contenant un plusieurs dérivés de 2,6-diamino toluène à titre de coupleur, en association avec une ou plusieurs bases d'oxydation sélectionnées parmi des bases d'oxydation pyrimidiniques et la 2-β-hydroxyéthyl parapfiénylénediamine, ces compositions pouvant également contenir à titre de base d'oxydation additionnelle, des dérivés de para-aminophénol comme le 3-méthyl para-aminophénol et le 2-méthyl para-aminophénol.

Cependant bien que les colorations obtenues en mettant en oeuvre de telles compositions soient très chromatiques, elles ne sont pas entièrement satisfaisantes, notamment du point de vue de leur ténacité vis à vis des différents traitements et agressions que peuvent subir les fibres kératiniques.

Or, la demanderesse vient maintenant de découvrir qu'il est possible d'obtenir de nouvelles teintures, capables de conduire à des colorations puissantes et très chromatiques, et résistant bien aux diverses agressions que peuvent subir les fibres, en associant au moins une base d'oxydation sélectionnée par des dérivés de para-aminophénols de formule (I) définie ci-après, et du 1,3-bis-(β-hydroxyéthyl)amino 2-méthyl benzène et/ou au moins l'un de ses sels d'addition avec un acide à titre de coupleur, ladite composition étant exempte de toute base d'oxydation qui serait choisie parmi la pyrimidine et ses dérivés, la 2-β-hydroxyéthyl paraphénylènediamine et leurs sels d'addition avec un acide.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les para-aminophénols substitués de formule (I) suivante, et leurs sels d'addition avec un acide :

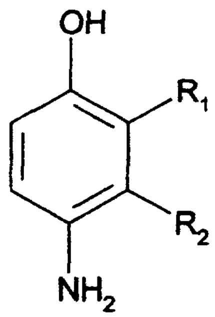

(I)

dans laquelle:

- $R_1$ représente un atome d'hydrogène ou de fluor, un radical alkyle en $C_1$-$C_4$, monohydroxyalkyle en $C_1$-$C_4$, polyhydroxyalkyle en $C_2$-$C_4$, alcoxyalkyle en $C_1$-$C_4$, aminoalkyle en $C_1$-$C_4$, ou monohydroxyalkyle($C_1$-$C_4$)aminoalkyle en $C_1$-$C_4$ ;
- $R_2$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxyalkyle en $C_1$-$C_4$, sous réserve qu' un seul, des radicaux $R_1$ et $R_2$ représente un atome d'hydrogène ;
- du 1,3-bis-(β-hydroxyéthyl)amino 2-méthyl benzène et/ou au moins l'un de ses sels d'addition avec un acide à titre de coupleur ;

ladite composition étant exempte de toute base d'oxydation additionnelle choisie parmi la pyrimidine et ses dérivés, la 2-β-hydroxyéthyl paraphénylènediamine, et leurs sels d'addition avec un acide.

La composition tinctoriale conforme à invention conduit à des colorations puissantes, très chromatiques, et présentant d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale.

Parmi les para-aminophénols substitués de formule (I) ci-dessus, on peut plus particulièrement citer, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Le ou les para-aminophénols substitués de formule (I) représentent de préférence de 0,0005 à 12 % en poids environ du poids total de la composition tinctoriale conforme à l'invention, et encore plus préférentiellement de 0,005 à 6 % en poids environ de ce poids.

Le 1,3-bis-(β-hydroxyéthyl)amino 2-méthyl benzène et/ou le ou ses sels d'addition avec un acide utilisés à titre de coupleur selon l'invention, représentent de préférence de 0,001 à 10 % en poids environ du poids total de la composition tinctoriale et encore plus préférentiellement de 0,01 à 5 % en poids environ de ce poids.

La composition tinctoriale conforme à l'invention peut en outre contenir un ou plusieurs coupleurs additionnels différents du 1,3-bis-(β-hydroxyéthyl)amino 2-méthyl benzène et de ses sels d'addition avec un acide et/ou un ou plusieurs colorants directs, notamment pour modifier les nuances ou les enrichir en reflets.

Parmi les coupleurs pouvant être présents à titre additionnel dans la composition tinctoriale conforme à l'invention, on peut notamment citer les méta-aminophénols, les méta-phénylènediamines, les métadiphénols, les coupleurs hétérocycliques, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également contenir, en plus du ou des para-aminophénols substitués de formule (I), une ou plusieurs bases d'oxydation additionnelles choisies parmi les bases d'oxydation classiquement utilisées pour la teinture d'oxydation des fibres kératiniques, et parmi lesquelles on peut citer les paraphénylènediamines à l'exclusion de la 2-β-hydroxyéthyl paraphénylènediamine et de ses sels d'addition avec un acide, les orthophénylènediamines, les bases doubles et les bases hétérocycliques non pyrimidiniques.

Lorsqu'elles sont présentes, la ou les bases d'oxydation additionnelles représentent de préférence de 0,0005 à 12 % en poids environ du poids total de la composition tinctoriale conforme à l'invention, et encore plus préférentiellement de 0,005 à 6 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates, les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (II) suivante :

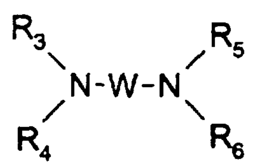

(II)

dans laquelle W est un reste propylène éventuellement substitué par un groupement hydroxyle ou un radical alkyle en $C_1$-$C_4$ ; $R_3$, $R_4$, $R_5$ et $R_6$, identiques ou différents, représentent un atome d'hydrogène, un radical alkyle en $C_1$-$C_4$ ou hydroxyalkyle en $C_1$-$C_4$.

La composition tinctoriale conforme à l'invention peut également renfermer divers adjuvants utilisés classiquement dans les compositions pour la teinture des cheveux.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydo-réductases à 2 électrons, les peroxydases et les laccases. Le peroxyde d'hydrogène est particulièrement préféré.

Le pH de la composition oxydante renfermant ragent oxydant tel que défini ci-dessus est tel qu'après mélange

avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

## EXEMPLES

### EXEMPLES DE TEINTURE 1 et 2 EN MILIEU ALCALIN

On a préparé les compositions tinctoriales suivantes (teneurs en grammes) :

| EXEMPLE | 1 | 2 |
|---|---|---|
| 3-méthyl 4-amino phénol (base d'oxydation de formule (I)) | 0,49 | - |
| 4-amino 2-(β-hydroxyéthyl aminométhyl) phénol, 2HCl (base d'oxydation de formule (I)) | - | 1,0 |
| 1,3-bis-(β-hydroxyéthyl)amino 2-méthyl benzène (coupleur) | 0,84 | 0,84 |
| Support de teinture commun | (*) | (*) |
| Eau déminéralisée qsp | 100 g | 100 g |

(*) : Support de teinture commun :
- Ethanol à 96° 18 g
- Métabisulfite de sodium en solution aqueuse à 35% 0,68 g
- Sel pentasodique de l'acide diéthylène triamino pentacétique 1,1 g
- Ammoniaque à 20% de $NH_3$ 10 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

Les cheveux ont été teints dans les nuances figurent dans le tableau ci-après :

| EXEMPLE | NUANCE OBTENUE |
|---|---|
| 1 | Rose |
| 2 | Violine chromatique |

### EXEMPLES 3 A 5 COMPARATIFS

On a préparé les compositions tinctoriales suivantes (teneurs en grammes) :

| EXEMPLE | 3(**) | 4 | 5 |
|---|---|---|---|
| Para-aminophénol (base d'oxydation ne faisant pas partie de l'invention) | 0,327 | - | - |
| 3-méthyl 4-amino phénol (base d'oxydation de formule (I)) | | 0,369 | - |

(**) : exemple comparatif ne faisant pas partie de l'invention.

(suite)

| EXEMPLE | 3(**) | 4 | 5 |
|---|---|---|---|
| 4-amino 3-fluoro phénol (base d'oxydation de formule (I)) | | - | 0,381 |
| 1,3-bis-(β-hydroxyéthyl)amino 2-méthyl benzène (coupleur) | 0,63 | 0,63 | 0,63 |
| Support de teinture commun n°2 | (***) | (***) | (***) |
| Eau déminéralisée qsp | 100 g | 100 g | 100 g |

(**) : exemple comparatif ne faisant pas partie de l'invention.

(***) : Support de teinture commun n°2 :
- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN 012 ® par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10,0 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux châtains décolorés.

Les cheveux ont ensuite été rincés à l'eau, lavés avec un shampooing standard, rincés à nouveau puis séchés.

Sur les mèches de cheveux ainsi teintes avec les compositions des exemples 3 à 5 ci-dessus, un test de résistance à 6 shampooings consécutifs a été réalisé.

La couleur des mèches de cheveux teintes a été évaluée dans le système MUNSELL au moyen d'un colorimètre CM 2002 MINOLTA, avant le test de résistance aux shampooings.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

Les mèches de cheveux teintes ont ensuite été soumises à un test de résistance aux shampooings. Les shampooings ont été réalisés dans une machine STAR, (Système de Traitement Automatisé et Robotique), qui enchaîne automatiquement les lavages, les rinçages et les séchages, et telle que décrite par exemple dans les demandes de brevet FR-A-2 751 720 et FR-A-2 752 460.

Pour ce faire, chaque mèches de cheveux a été enroulée dans un godet dont le fond a été garni d'un tampon en mousse. Chaque mèche de cheveux a été soumise 6 fois au cycle suivant :

- Mouillage à l'eau pendant 1 minute;
- Essorage pendant 30 secondes ;
- Shampouinage pendant 2 minutes et 30 secondes (avec du shampooing DOP ® à la Camomille dilué à 2%) ;
- Rinçage avec de l'eau à 33°C pendant 2 minutes ;
- Séchage pendant 25 minutes.

Les mèches teintes ont ainsi été soumises à 6 épreuves de shampooing consécutives.

La couleur des mèches a été ensuite évaluée à nouveau dans le système MUNSELL au moyen d'un colorimètre CM 2002 MINOLTA.

La différence entre la couleur de la mèche avant les shampooings et la couleur de la mèche après les shampooings a été calculée en appliquant la formule de NICKERSON :

$$\Delta E = 0,4\ C_0 \Delta H + 6 \Delta V + 3\ \Delta C$$

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et $C_0$ représente la pureté de la mèche par rapport à laquelle on désire évaluer la différence de couleur, c'est à dire la pureté de la mèche avant le test de résistance aux shampooings.

La dégradation de la couleur (ΔE) est d'autant plus importante que le chiffre indiqué est élevé.

Les résultats obtenus figurent dans le tableau ci-après :

| EXEMPLE | Couleur des cheveux avant les shampooings | Couleur des cheveux après les shampooings | Dégradation de la couleur | | | |
|---|---|---|---|---|---|---|
| | | | ΔH | ΔV | ΔC | **ΔE** |
| **3 (**)** | 0.6 R 3.1/5.8 | 7.8 R 4.1 / 4.1 | 7.2 | 1.0 | 1.7 | **27.8** |
| **4** | 5.9 R 3.9 / 5.1 | 0.8 YR 4.5 / 4.2 | 4.9 | 0.6 | 0.9 | **16.3** |
| **5** | 4.4 R 3.8 / 5.0 | 0.3 YR 4.3/4.1 | 5.9 | 0.5 | 0.9 | **17.5** |

(**) : exemple comparatif ne faisant pas partie de l'invention.

Ces résultats montrent que la composition de l'exemple 3 ne faisant pas partie de l'invention et telle que décrite par exemple dans la demande de brevet DE 4 132 615, contenant l'association du 1,3-bis-(β-hydroxyéthyl) amino 2-méthyl benzène à titre de coupleur et du para-aminophénol à titre de base d'oxydation, conduit à une coloration résistant beaucoup moins bien à l'action des shampooings que les colorations obtenues en mettant en oeuvre les compositions des exemples 4 ou 5 conformes à l'invention, c'est à dire contenant l'association du même coupleur mais avec un para-aminophénol substitué.

## Revendications

**1.** Composition pour la teinture d'oxydation des fibres kératiniques et en particulier des fibres kératiniques humaines telles que les cheveux, **caractérisée par le fait qu'**elle comprend, dans un milieu approprié pour la teinture :

- au moins une base d'oxydation choisie parmi les para-amlnophénols substitués de formule (I) suivante et leurs sels d'addition avec un acide :

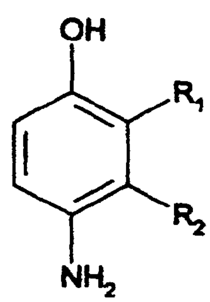

(I)

dans laquelle :

- $R_1$ représente un atome d'hydrogène ou de fluor, un radical alkyle en $C_1$-$C_4$, monohydroxyalkyle en $C_1$-$C_4$, polyhydroxyalkyle en $C_2$-$C_4$, alcoxyalkyle en $C_1$-$C_4$, aminoalkyle en $C_1$-$C_4$, ou monohydroxyalkyle($C_1$-$C_4$)aminoalkyle en $C_1$-$C_4$ ;
- $R_2$ représente un atome d'hydrogène ou de fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxyalkyle en $C_1$-$C_4$, sous réserve qu' un seul, des radicaux $R_1$ et $R_2$ représente un atome d'hydrogène ;
- du 1,3-bis-(β-hydroxyéthyl)amino 2-méthyl benzène et/ou au moins l'un de ses sels d'addition avec un acide à titre de coupleur ;

ladite composition étant exempte de base d'oxydation additionnelle choisie parmi la pyrimidine et ses dérivés, la 2-β-hydroxyéthyl paraphénylènediamine, et leurs sels d'addition avec un acide.

2. Composition selon la revendication 1, **caractérisée par le fait que** le ou les para-aminophénols substitués de formule (I) sont choisis parmi le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

3. Composition selon la revendication 1 ou 2, **caractérisée par le fait que** le ou les para-aminophénols substitués de formule (I) représentent de 0,0005 à 12 % en poids du poids total de la composition tinctoriale.

4. Composition selon la revendication 3, **caractérisée par le fait que** le ou les para-aminophénols substitués de formule (I) représentent de 0,005 à 6 % en poids du poids total de la composition tinctoriale.

5. Composition selon l'une quelconque des revendications précédentes, **caractérisée par le fait que** le 1,3-bis-(β-hydroxyéthyl)amino 2-méthyl benzène et/ou le ou ses sels d'addition avec un acide représentent de 0,001 à 10 % en poids du poids total de la composition tinctoriale.

6. Composition selon la revendication 5, **caractérisée par le fait que** le 1,3-bis-(β-hydroxyéthyl)amino 2-méthyl benzène et/ou le ou ses sels d'addition avec un acide représentent de 0,01 à 5 % en poids du poids total de la composition tinctoriale.

7. Composition selon l'une quelconque des revendications précédentes, **caractérisée par le fait qu'**elle renferme un ou plusieurs coupleurs additionnels différents du 1,3-bis-(β-hydroxyéthyl)amino 2-méthyl benzène et de ses sels d'addition avec un acide et/ou un ou plusieurs colorants directs.

8. Composition selon l'une quelconque des revendications précédentes, **caractérisée par le fait que** les sels d'addition avec un acide sont choisis parmi les chlorhydrates, les bromhydrates, les sulfates, les tartrates, les lactates et les acétates.

9. Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 8, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

10. Procédé selon la revendication 9, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

11. Disposer à plusieurs compartiments, ou "kit" de teinture à plusieurs compartiments, dont un premier compartiment renferme une composition tinctoriale telle que définie à l'une quelconque des revendications 1 à 8 et un second compartiment renferme une composition oxydante.

**Patentansprüche**

1. Zusammensetzung zum oxidativen Färben von Keratinfasern und insbesondere menschlichen Keratinfasern, wie Haaren, **dadurch gekennzeichnet, dass** sie in einem zum Färben geeigneten Medium enthält:

- mindestens eine Oxidationsbase, die unter den substituierten p-Aminophenolderivaten der folgenden Formel (I) und deren Additionssalzen mit einer Säure ausgewählt ist:

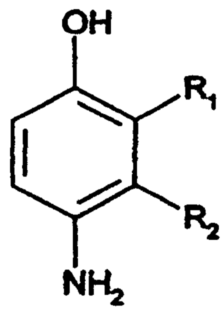

(I)

worin bedeuten:

- $R_1$ Wasserstoff, Fluor, Alkyl($C_{1-4}$), Monohydroxyalkyl($C_{1-4}$), Polyhydroxyalkyl($C_{2-4}$), Alkoxyalkyl($C_{1-4}$), Aminoalkyl($C_{1-4}$) oder Monohydroxyalkyl($C_{1-4}$)aminoalkyl($C_{1-4}$);
- $R_2$ Wasserstoff, Fluor, Alkyl($C_{1-4}$), Monohydroxyalkyl($C_{1-4}$), Polyhydroxyalkyl($C_{2-4}$), Aminoalkyl($C_{1-4}$), Cyanoalkyl($C_{1-4}$) oder Alkoxyalkyl($C_{1-4}$),

mit der Maßgabe, dass nur eine der Gruppen $R_1$ oder $R_2$ ein Wasserstoffatom bedeutet;

- als Kuppler das 1,3-Bis(β-hydroxyethyl)amino-2-methyl-benzol und/oder mindestens eines seiner Additionssalze mit einer Säure,

wobei die Zusammensetzung keine weitere Oxidationsbase enthält, die unter Pyrimidin und seinen Derivaten, 2-β-Hydroxyethyl-p-phenylendiamin und deren Additionssalzen mit einer Säure ausgewählt ist.

**2.** Zusammensetzung nach Anspruch 1, **dadurch gekennzeichnet, dass** das oder die substituierte(n) p-Aminophenol(e) der Formel (I) unter 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)phenol, 4-Amino-2-fluor-phenol und den Additionssalze dieser Verbindungen mit einer Säure ausgewählt sind.

**3.** Zusammensetzung nach Anspruch 1 oder 2, **dadurch gekennzeichnet, dass** das oder die substituierte(n) p-Aminophenol(e) der Formel (I) 0,0005 bis 12 Gew.-% des Gesamtgewichts der Farbmittelzusammensetzung ausmachen.

**4.** Zusammensetzung nach Anspruch 3, **dadurch gekennzeichnet, dass** das oder die substituierte(n) p-Aminophenol(e) der Formel (I) 0,005 bis 6 Gew.-% des Gesamtgewichts der Farbmittelzusammensetzung ausmachen.

**5.** Zusammensetzung nach einem der vorhergehenden Ansprüche, **dadurch gekennzeichnet, dass** das 1,3-Bis(β-hydroxyethyl)amino-2-methyl-benzol und/oder sein(e) Additionssalz(e) mit einer Säure 0,001 bis 10 Gew.-% des Gesamtgewichts der Farbmittelzusammensetzung ausmachen.

**6.** Zusammensetzung nach Anspruch 5, **dadurch gekennzeichnet, dass** das 1,3-Bis(β-hydroxyethyl)amino-2-methyl-benzol und/oder sein(e) Additionssalz(e) mit einer Säure 0,01 bis 5 Gew.-% des Gesamtgewichts der Farbmittelzusammensetzung ausmachen.

**7.** Zusammensetzung nach einem der vorhergehenden Ansprüche, **dadurch gekennzeichnet, dass** sie einen oder mehrere zusätzliche Kuppler, die von dem 1,3-Bis(β-hydroxyethyl)amino-2-methyl-benzol und seinen Additionssalzen mit einer Säure verschieden sind, und/oder einen oder mehrer Direktfarbstoffe enthält.

**8.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**9.** Verfahren zum Färben von Keratinfasern und insbesondere menschlichen Keratinfasern, beispielsweise zum Färben der Haare, **dadurch gekennzeichnet, dass** auf die Fasern mindestens eine Farbmittelzusammensetzung nach einem der Ansprüche 1 bis 8 aufgebracht wird und die Farbe bei einem sauren, neutralen oder alkalischen pH-Wert mit einem Oxidationsmittel entwickelt wird, das unmittelbar bei der Anwendung zu der Farbmittelzusammensetzung gegeben wird oder das in einer oxidierenden Zusammensetzung vorliegt, die gleichzeitig oder getrennt davon anschließend aufgebracht wird.

**10.** Verfahren nach Anspruch 9, **dadurch gekennzeichnet, dass** das in der oxidierenden Zusammensetzung vorliegende Oxidationsmittel unter Wasserstoffperoxid, Harnstoffperoxid, Alkalimetallbromaten, Salzen von Persäuren, wie Perboraten, Percarbonaten und Persulfaten, Persäuren und Enzymen ausgewählt ist.

**11.** Vorrichtung mit mehreren Abteilungen oder "Kit" zum Färben mit mehreren Abteilungen, **dadurch gekennzeichnet, dass** eine erste Abteilung die in den Ansprüchen 1 bis 8 definierte Farbmittelzusammensetzung und eine zweite Abteilung eine oxidierende Zusammensetzung enthält.

## Claims

**1.** Composition for the oxidation dyeing of keratin fibres, and in particular human keratin fibres such as the hair, **characterized in that** it comprises, in a medium which is suitable for dyeing:

- at least one oxidation base chosen from the substituted para-aminophenols of formula (I) below, and the addition salts thereof with an acid:

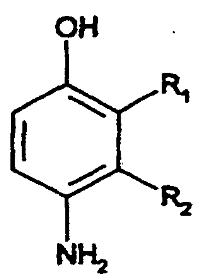

(I)

in which:

- $R_1$ represents a hydrogen or fluorine atom, a $C_1$-$C_4$ alkyl radical, a $C_1$-$C_4$ monohydroxyalkyl radical, a $C_2$-$C_4$ polyhydroxyalkyl radical, a $C_1$-$C_4$ alkoxyalkyl radical, a $C_1$-$C_4$ aminoalkyl radical or a monohydroxy($C_1$-$C_4$)alkylamino($C_1$-$C_4$)alkyl radical;
- $R_2$ represents a hydrogen or fluorine atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical, a cyano($C_1$-$C_4$)alkyl radical or a $C_1$-$C_4$ alkoxyalkyl radical, with the proviso that only one of the radicals $R_1$ and $R_2$ represents a hydrogen atom;
- 1,3-bis(β-hydroxyethyl)amino-2-methylbenzene and/or at least one of the addition salts thereof with an acid, as coupler;

the said composition being free of additional oxidation base chosen from pyrimidine and derivatives thereof, 2-β-hydroxyethyl-para-phenylenediamine, and the addition salts thereof with an acid.

**2.** Composition according to Claim 1, **characterized in that** the substituted para-aminophenol(s) of formula (I) is (are) chosen from 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

**3.** Composition according to Claim 1 or 2, **characterized in that** the substituted para-aminophenol(s) of formula (I) represent(s) from 0.0005 to 12% by weight relative to the total weight of the dye composition.

**4.** Composition according to Claim 3, **characterized in that** the substituted para-aminophenol(s) of formula (I) represent(s) from 0.005 to 6% by weight relative to the total weight of the dye composition.

**5.** Composition according to any one of the preceding claims, **characterized in that** the 1,3-bis(β-hydroxyethyl) amino-2-methylbenzene and/or the addition salt(s) thereof with an acid represent(s) from 0.001 to 10% by weight relative to the total weight of the dye composition.

**6.** Composition according to Claim 5, **characterized in that** the 1,3-bis(β-hydroxyethyl)amino-2-methylbenzene and/or the addition salt(s) thereof with an acid represent(s) from 0.01 to 5% by weight relative to the total weight of the dye composition.

**7.** Composition according to any one of the preceding claims, **characterized in that** it contains one or more additional couplers other than 1,3-bis(β-hydroxyethyl)amino-2-methylbenzene and other than the addition salts thereof with an acid, and/or one or more direct dyes.

**8.** Composition according to any one of the preceding claims, **characterized in that** the addition salts with an acid are chosen from the hydrochlorides, hydrobromides, sulphates, tartrates, lactates and acetates.

**9.** Process for dyeing keratin fibres, and in particular human keratin fibres such as the hair, **characterized in that** at least one dye composition as defined in any one of Claims 1 to 8 is applied to the said fibres, and **in that** the colour is developed at acidic, neutral or alkaline pH with the aid of an oxidizing agent which is added to the dye composition only at the time of use, or which is present in an oxidizing composition that is applied simultaneously or sequentially.

**10.** Process according to Claim 9, **characterized in that** the oxidizing agent present in the oxidizing composition is chosen from hydrogen peroxide, urea peroxide, alkali metal bromates, persalts such as perborates, percarbonates and persulphates, peracids and enzymes.

**11.** Multi-compartment dyeing device or multicompartment dyeing kit, a first compartment of which contains a dye composition as defined in any one of Claims 1 to 8 and a second compartment of which contains an oxidizing composition.